Europäisches Patentamt

**European Patent Office**

**Office européen des brevets**

(11) Veröffentlichungsnummer: **0 290 746**

**A1**

(12) **EUROPÄISCHE PATENTANMELDUNG**

(21) Anmeldenummer: **88104070.3**

(22) Anmeldetag: **15.03.88**

(51) Int. Cl.⁴: **B01F 15/00**

(30) Priorität: **14.05.87 CH 1852/87**

(43) Veröffentlichungstag der Anmeldung:
**17.11.88 Patentblatt 88/46**

(84) Benannte Vertragsstaaten:
**AT BE DE ES FR IT NL SE**

(71) Anmelder: **ALLO PRO AG**
**Grabenstrasse 25**
**CH-6340 Baar(CH)**

(72) Erfinder: **Ziemann, Edeltraud**
**Längenthalerstrasse 9**
**D-8252 Inning am Holz(DE)**
Erfinder: **Ziemann, Andreas**
**Müllerstrasse 26**
**D-8058 Erding(DE)**

(74) Vertreter: **Dipl.-Ing. H. Marsch Dipl.-Ing. K.**
**Sparing Dipl.-Phys.Dr. W.H. Röhl**
**Patentanwälte**
**Rethelstrasse 123**
**D-4000 Düsseldorf(DE)**

(54) **Gerät zum Steuern und Überwachen der Zeitintervalle beim Mischen und Verarbeiten von Knochenzement.**

(57) Ein Steuerelement (17) steuert die Mischung der Komponenten des Knochenzementes unter Vakuum und überwacht die Zeitintervalle für die Ruhe-, Verarbeitungs-und Aushärtezeit.

Die temperaturabhängige Reaktionsdauer der Polymerisationsreaktion kann bei der Länge einzelner Zeitintervalle dadurch berücksichtigt werden, dass durch das Steuerelement (17) Messwerte eines Messfühlers (18) für die Umgebungstemperatur in einem begrenzten Temperaturbereich berücksichtigt werden.

Mit dem neuen Gerät (1) ergeben sich Knochenzement-Mischungen mit hohen Festigkeiten und geringer Porosität, wobei die Eigenschaften des Zementes darüberhinaus gut reproduzierbar sind.

Fig. 3

## Gerät zum Steuern und Ueberwachen der Zeitintervalle beim Mischen und Verarbeiten von Knochenzement

Die Erfindung betrifft ein Gerät zum Steuern und Ueberwachen der Zeitintervalle beim Mischen und Verarbeiten von Knochenzement, welches Gerät eine Mischvorrichtung mit mindestens einem als Spritzenzylinder ausgebildeten Mischbehälter und, durch je einen Motor angetrieben, einen Rührer sowie eine Vakuumpumpe enthält.

Knochenzemente werden bekanntlich im allgemeinen durch Mischen von flüssigem Methylmethacrylat-Monomer und pulverförmigem Polymethylmethacrylat - unter Zugabe von Härtern und Beschleunigern - hergestellt, wobei das Aushärten der flüssigen Komponente die zuvor teigartige Mischung verfestigt. Das Mischen der Komponenten erfolgt dabei von Hand mit Spateln oder rotierenden Rührern, die gegebenenfalls auch von einem Motor angetrieben sein können (siehe z.B. F.J.Kummer "Improved Mixing of Bone Cement", Seite 238 des Tagungsberichts vom 31st Annual ORS, Las Vegas, Nevada, USA, vom 21. bis 24. Januar 1985). Weiterhin ist es bekannt, ein kontrolliertes Vakuum in dem Mischgefäss zu erzeugen und aufrechtzuerhalten, um das Gemisch möglichst weitgehend zu entgasen (siehe z.B. R.L.Wixon et al "Vacum Mixing of Methylmethacrylate Bone Cements", Seite 327 des gleichen Tagungsberichtes). Nach Beendigung des Mischvorganges ergibt sich bei der Verarbeitung von niedrigviskosen Zementen, die vorzugsweise mit Hilfe einer Zementspritze erfolgen kann, zunächst ein Zeitntervall, die sogenannte Ruhezeit, in dem die Polymerisation so weit fortschreitet, dass das teigartige Gemisch eine Konsistenz erhält, in der es verarbeitbar ist und die Monomerabgabe reduziert wird. An diese Ruhezeit schliesst sich ein Zeitintervall für die Verarbeitung und eines für die Aushärtung des Zementes an.

Die auf die geschilderte Art hergestellten Knochenzemente haben sich in ihren Eigenschaften - z.B. in ihrer Festigkeit oder ihrer Porosität - als wenig reproduzierbar erwiesen.

Aufgabe der Erfindung ist es daher, ein Gerät zu schaffen, mit dem die Eigenschaften des Knochenzementes von den individuellen Einflüssen der mischenden Personen unabhängig und infolgedessen reproduzierbar werden. Gleichzeitig soll mit dem neuen Gerät selbstverständlich eine Verbesserung der mechanischen Eigenschaften des ausgehärteten Knochenzementes erreicht werden.

Diese Aufgabe wird erfindungsgemäss gelöst durch ein Steuerelement, das über einen Zeitzähler den Ablauf des Mischens steuert und mindestens einzelne Zeitintervalle beim Verarbeiten des Knochenzementes überwacht, wobei mindestens das Ende des Mischens und mindestens einzelner überwachter Zeitintervalle durch akustische und/oder optische Signale angezeigt wird.

Die mit dem neuen Gerät erreichte Steuerung der - bei konstanter Umgebungstemperatur gleichbleibenden - Zeitintervalle für die einzelnen Misch- und Verarbeitungs-"Schritte" des Zementes bringt eine Verbesserung und gute Reproduzierbarkeit der mechanischen Eigenschaften.

Da die chemische Reaktionszeit - und damit die Zeitintervalle der Ruhezeit, der Verarbeitungszeit und der Aushärtungszeit - von Knochenzementen bekanntlich temperaturabhängig ist, ist es vorteilhaft, wenn ein Messfühler für die Umgebungstemperatur vorhanden ist, der die Dauer einzelner dieser Zeitintervallen temperaturabhängig verändert.

Weiterhin besteht selbstverständlich bei dem neuen Gerät eine Möglichkeit, die Dauer mindestens der temperaturabhängigen Zeitintervalle für unterschiedlich zusammengesetzte Knochenzemente zu verändern.

Um ein Eindringen des Rührers in die teigartige Mischung zu erleichtern, ist es zweckmässig, zunächst ein Anmischen mit relativ niedriger Rührgeschwindigkeit - von etwa 40 U/min bis 100 U/min - durchzuführen und nach dem Anmischen die Rührgeschwindigkeit auf 120 U/min bis 300 U/min zu erhöhen. Hierfür kann der Rührermotor von einer niedrigen auf eine höhere Rührgeschwindigkeit umschaltbar sein.

Weiterhin kann die Durchmischung des Zementes gefördert werden, wenn der Rührermotor in seiner Drehrichtung umschaltbar ist.

Wie bereits erwähnt, ist es bekannt, dass die Qualität von Knochenzementen erheblich verbessert werden kann, wenn das Mischen des flüssigen Monomers mit der Pulverkomponente unter Vakuum durchgeführt wird, wobei für dieses mit Vorteil eine Druckdifferenz von etwa 500 mbar gegen den Umgebungsdruck nicht überschritten werden soll. Grössere Druckdifferenzen bewirken ein starkes Verdampfen des Monomers; bei erheblich geringeren Vakua ist dagegen das Ausgasen ungenügend. Es ist daher vorteilhaft, wenn das Vakuum durch einen Drucksensor auf einer konstanten Druckdifferenz zum Umgebungsdruck gehalten ist.

Ein wesentliches Element für eine gute und vollständige Durchmischung der Komponenten ist der Rührer. Experimentell hat sich hier eine Konstruktion als besonders wirksam erwiesen, bei der der Rührer eine den Spritzzylinder in axialer Richtung mindestens teilweise ausfüllende, korkenzieherähnliche Form hat, deren Aussendurchmesser an den Innendurchmesser des Mischzylinders angepasst ist. Um am Boden des kartuschenartigen Spritzenzylinders besonders in der Randzone befindliche Gemisch wieder in den Mischprozess zurückzuführen, kann der Rührer mit Vorteil an seinem freien Ende in einen horizontalen Aufnehmer übergehen, der V-förmig die Mittelachse des Zylinders umfasst. Bei dieser "Rückführung" kann eine sich mit dem Rührer mitdrehende Gemischsäule entstehen. Diese wird vorteilhafterweise immer wieder zerstört durch einen in den Korkenzieher hinein gerichteten Abreisshaken, der an den horizontalen Aufnehmer angesetzt ist.

Weitere Verbesserungen bei dem Mischen des Knochenzementes können erzielt werden, wenn der Abreisshaken mindestens annähernd mit dem ihn erzeugenden V-Arm in einer zur Rührerachse parallelen Ebene verläuft, und/oder wenn der Abreisshaken aus der Horizontalen heraus unter einem Winkel von mindestens annähernd 60° nach oben gerichtet ist; schliesslich haben die Versuche ergeben, dass es günstig ist, wenn der Abreisschaken vor der achsparallelen Mittelebene des Rührers endet.

Weiterhin sind in dem Gerät über die beanspruchten Funktionen hinaus Möglichkeiten vorgesehen, die vollautomatischen Abläufe der Zeitintervalle durch manuelle Eingaben anderer Zeitintervalle - beispielsweise beim Testen neuer Zementmischungen - gegebenenfalls zu ändern. Darüberhinaus werden - neben dem Ablauf der einzelnen Programm-Schritte, die im allgemeinen akustisch und/oder optisch angezeigt werden - Fehler im Gerät oder unzulässige Grenzwertüberschreitungen bei Temperatur und/oder Druck, die beispielsweise vom Temperaturfühler oder vom Drucksensor ermittelt werden, ebenfalls akustisch und/oder optisch signalisiert.

Im folgenden wird die Erfindung anhand eines Ausführungsbeispiels im Zusammenhang mit der Zeichnung näher erläutert.

Fig. 1 und 2 stellen eine Vorderansicht sowie eine Draufsicht auf das neue Gerät dar;

Fig. 3 ein schematisches Blockdiagramm, auf dem das Steuerelement und die es beeinflussenden Messfühler bzw. die von ihm beeinflussten Funkionselemente wiedergegeben sind;

Fig. 4 gibt in einem schematischen Diagramm den Ablauf der von den Gerät gesteuerten und überwachten Herstellungs-und Verarbeitungsschritte V für einen ausgewählten Knochenzement in Abhängigkeit von der Zeit t wieder, wobei für temperaturbeeinflusste Schritte als Parameter die Temperatur T berücksichtigt ist;

Fig. 5 ist eine Ansicht des für das Gerät konstruierten korkenzieherähnlichen Rührers;

Fig. 6 ist·ein Schnitt VI-VI von Fig. 5.

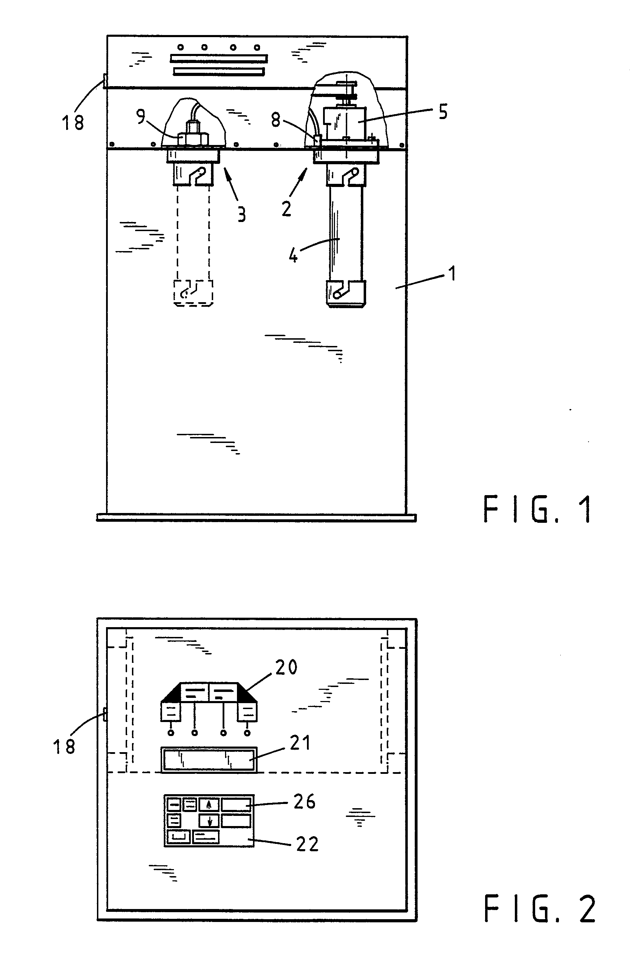

In seiner als Ausführungsbeispiel gewählten Form hat das Gerät 1 (Fig. 1) zwei Anschlussstellen 2 und 3 für den Anschluss der als Spritzenzylinder ausgebildeten Kartusche 4. Die rechte Anschlussstelle 2 dient dabei als Mischstation, während die linke 3 als Ruhe-oder Evakuierstation vorgesehen ist.

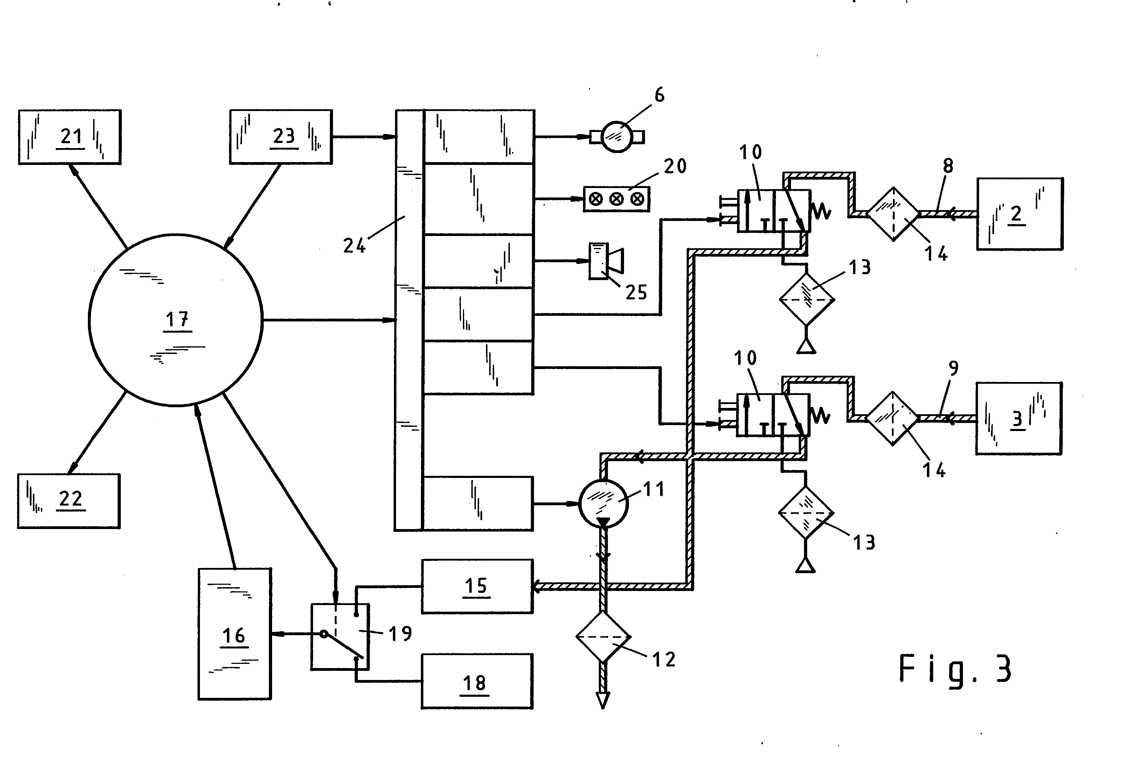

Entsprechend der verschiedenen Aufgaben ist die rechte Anschlussstelle 2 mit einem Rührwerk 5 ausgerüstet, das von einem Motor 6 (Fig. 3) angetrieben ist. Dieser kann sowohl in seiner Drehrichtung umschaltbar sein als auch mindestens für eine Drehrichtung mindestens zwei unterschiedliche Drehgeschwindigkeiten besitzen, eine niedrige von beispielsweise 40 bis 100 U/min für das sogenannte Anmischen und eine relativ dazu erhöhte Drehgeschwindigkeit von beispielsweise 120 bis 300 U/min für den eigentlichen Mischvorgang. Die optimale Dauer des Mischvorganges kann für verschiedene Knochenzement-Zusammensetzungen unterschiedlich sein; sie ist daher am Gerät einstellbar.

Beide Kartuschen-Anschlussstellen 2 und 3 sind mit Leitungsanschlüssen 8 und 9 versehen, die über Umschaltventile 10 (Fig. 3) mit einer Vakuumpumpe 11 (Fig. 3) verbunden sind.

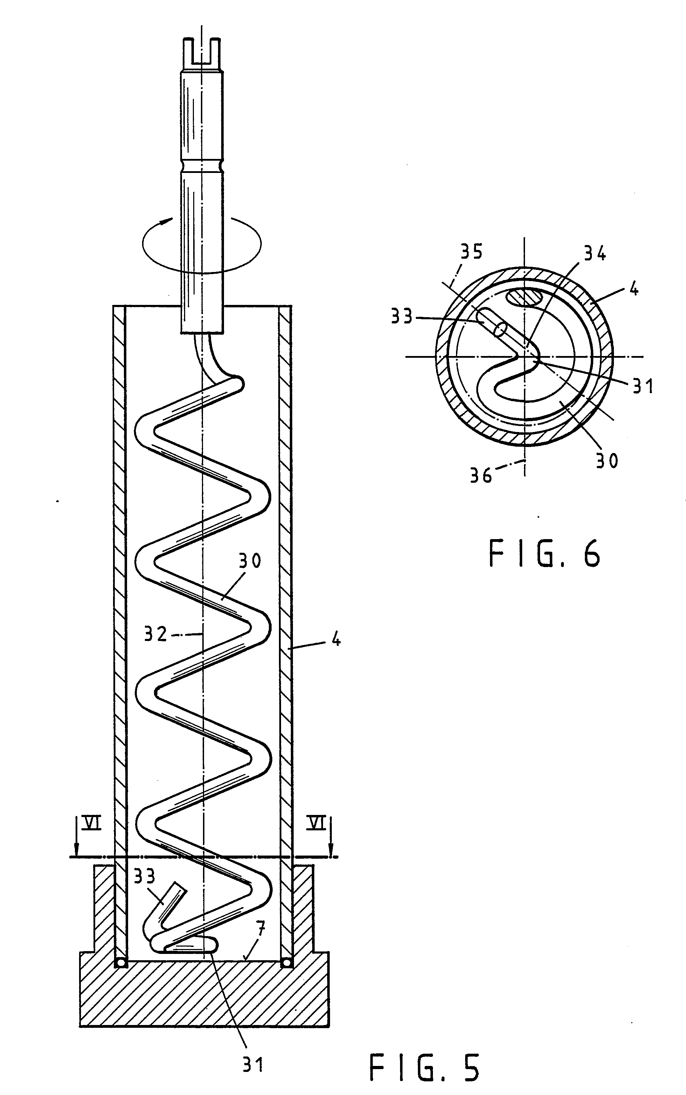

Das Vorhandensein von zwei Anschlussstellen ist jedoch keine zwingende Notwendigkeit; es kann auch nur eine Anschlusstelle vorhanden sein, die als Misch-und Ruhestation dient, wobei für die "Ruhe"-Funktion die Kartusche 4 kurz gelöst und der Rührer 30 (Fig. 5) aus dem Rührwerk 5 entfernt werden, sowie der Motor 6 abgeschaltet bleibt.

Die Umschaltventile 10 haben die Aufgabe, entweder die Pumpe 11 oder die umgebende Atmosphäre mit der Kartusche 4 zu verbinden, um in dieser wahlweise ein relatives Vakuum von etwa 500 mbar gegenüber dem Umgebungsdruck zu erzeugen und aufrechtzuerhalten oder die Kartusche 4 zu belüften.

Die von der Pumpe 11 abgesaugten Gase aus der Kartusche 4, die einen gewissen Prozentsatz verdampftes Monomer enthalten, werden über ein Kohlefilter 12 in die Umgebung ausgeblasen.

Um beim Aufheben des Vakuums in der Kartusche 4 die Sterilität zu wahren, sind in den Strömungswegen für die in die Kartusche 4 einströmende Luft vor den Umschaltventilen 10 Bakterien und Keime zurückhaltende Filter 13 vorgesehen. Schliesslich kann durch das Vakuum angesaugtes Polymerpulver durch Grobfilter 14 mit 2 um Porengrösse zurückgehalten werden.

Die Einhaltung eines Unterdruckes von etwa 500 mbar wird druch eine Druckdifferenz-Messfühler 15 (Fig. 3) gewährleistet, dessen Messignal über einen Messstellen-Umschalter 19 und einen Analog-Digital-Wandler 16 einem zentralen Steuerelement 17 zugeführt wird.

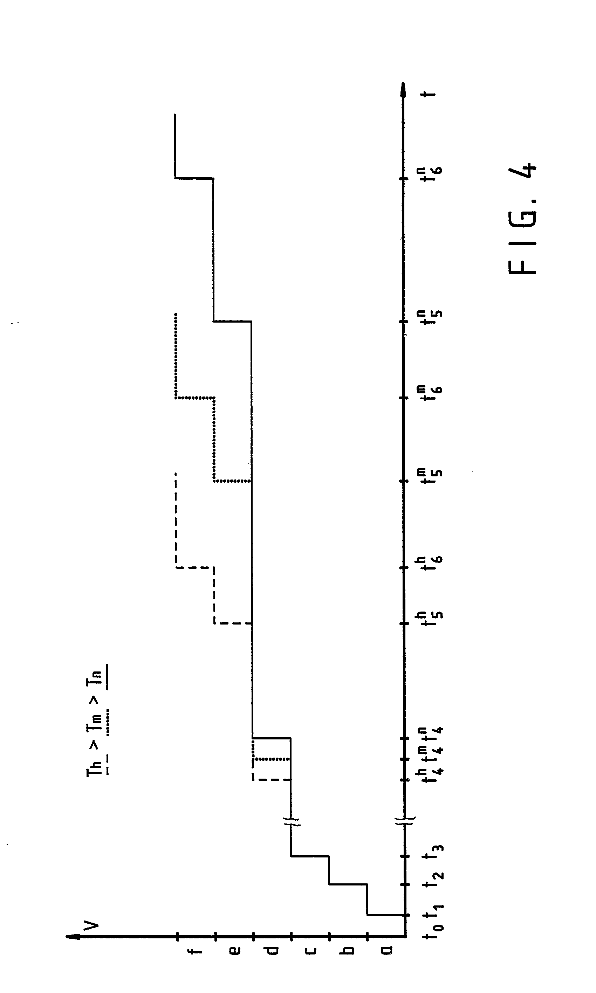

Dieses als auf dem Markt erhältlicher Mikroprozessor realisierte Steuerelement 17 steuert alle "Verfahrens"-Schritte V (Fig. 4), die für die Mischung und Verarbeitung des Knochenzementes notwendig sind, was im einzelnen noch beschrieben wird.

Ein zweiter Messfühler 18 (Fig. 3), dessen Messwerte ebenfalls über den Umschalter 19 und den Wandler 16 an das Steuerelement 17 gelangen, ist in einer Seitenwand des Gerätes 1 (Fig. 1) eingebaut. Er misst die Umgebungstemperatur, die gleichzeitig die Verarbeitungstemperatur für den Knochenzement ist. Bekanntlich hat diese Temperatur einen erheblichen Einfluss auf die Intervalle, die nach dem Mischen für die Verarbeitungs-"Reife", die Verarbeitung selbst und die Aushärtung zur Verfügung stehen.

Auf der Oberseite des Gerätes 1 (Fig. 2) sind für die optische Signalanzeige vier Leuchtdioden 20 (Fig. 3) vorgesehen, durch die vom Steuerelement 17 gesteuert verschiedene Betriebszustände des Gerätes 1 - z.B. "Gerät eingeschaltet", "automatisches Programm läuft", "manuelles Programm" oder "Gerät defekt" - signalisiert werden.

Weiterhin ist eine zweizeilige Schrifttafel 21 für Anweisungen an den Bediener des Gerätes 1 während des Ablaufes eines automatischen oder manuellen Programms oder für die Anzeige der bis zum Ablauf einzelner Zeitintervalle zur Verfügung stehenden Restzeiten vorhanden.

Schliesslich ist in die Oberseite des Gerätes 1 ein Eingabentastenfeld 22 eingebaut, das mit dem Steuerelement 17 in Wechselwirkung steht. Dieses Tastenfeld 22 einhält beispielsweise eine Starttaste 26 und eine Stopptaste, mit denen automatische oder manuelle Programme gestartet oder gestoppt werden können. Mit einer weiteren Taste kann die bei der gegebenen Temperatur und dem gewählten Zement zur Verfügung stehenende bzw. benötigte Gesamtzeit bis zur Aushärtung des Zementes ermittelt und in der Anzeige 21 sichtbar gemacht werden. Eine vierte Taste macht die gegebene Umgebungstemperatur und die bei dieser Temperatur bis zum Beginn der Verarbeitung notwendige Ruhezeit sichtbar.

Eine Mode-Taste schliesslich erlaubt die Auswahl verschiedener Funktionen, die mit weiteren Tasten ausgelöst werden. Unter Mode-Auswahl sind dabei eine " + " ("Pfeil rauf"), eine "-" ("Pfeil runter")-Eingabetaste und eine Abbruchtaste aktivierbar, die verschiedene Auswahl-und Funktionsänderungsmöglichkeiten eröffnet.

Im Inneren des Gerätes ist als akustischer Signalgeber, der beispielsweise "Vorwarnungen" vor Ablauf bestimmter Zeitintervalle gibt, ein elektronisches "Piep"-Element 25 (Fig. 3) vorhanden.

Wie bereits erwähnt, erfolgt die Steuerung aller Funktionen und Möglichkeiten des Gerätes durch den Mikroprozessor oder das Steuerelement 17, das, ebenso wie die energieverbrauchenden Elemente, von einem Netzteil 23 mit elektrischer Energie versorgt wird. Die Versorgung der Leistungsverbraucher ist in Fig. 3 durch eine Verteilerschiene 24 angedeutet, über die in der Darstellung auch die Verteilung der Steuerbefehle des Steuerelementes 17 auf den einzelnen Funktionselemente erfolgt.

In Fig. 3 sind Signal-Verbindungen von und zum Steuerlement 17 mit einfach durchgezogenen Linien dargestellt, wobei die Signalrichtung durch Pfeile angedeutet ist.

Anhand von Fig. 4, die in einem Diagramm den Ablauf der Verfahrensschritte V eines automatischen Programmes in Abhängkeit von der Zeit t zeigt, sei nun die Funktionsweise des Gerätes 1 für einen normalen Misch-und Verarbeitungsprozess erläutert, wobei temperaturabhängige Zeitabläufe für eine relative hohe Temperatur Th von etwa 24°, eine mittlere Temperatur Tm von etwa 21° und eine niedrige Umgebungstemperatur Tn von etwa 18° dargestellt sind. Bei dem beschriebenen zu mischenden und zu verarbeitenden Knochenzement handelt es sich um das unter dem Namen "Sulfix" (Warenzeichen der Anmelderin) bekannte Produkt.

Nach Einfüllen der pulverförmigen und der flüssigen Komponente des Zementes in eine Kartusche 4 wird durch Drücken der Starttaste 26 im Eingabentastenfeld 22 zur Zeit to das Gerät 1 in Betrieb gesetzt, das zur Angleichung seiner Temperatur an die Raumtemperatur des Operationsraumes längere Zeit - beispielsweise 1/2 bis 1 Stunde - zuvor aufgestellt worden ist; an der Mischstation 2 ist zur Zeit to ein Rührer 30 (Fig. 5) angesetzt. Mit dem Start des Gerätes 1 dreht sich der Rührer 30, dessen Motor zunächst mit einer Langsamgeschwindigkeit von 60 U/min läuft, aufgrund seiner Korkenzieherform langsam in das teigartige Gemisch aus flüssigem Monomer und kompakter Pulverkomponente. Bei langsam in die Kartusche 4 vordringendem Rührer 30 wird diese von dem Bediener des Gerätes nach oben geführt und an die Mischstation 2 angeschlossen. Dies ist der mit a bezeichnete Schritt in Fig. 4.

Nach Ablauf einer Zeitdifferenz von etwa 15 sec zum Zeitpunkt t1 ist das langsam laufende Anmischen beendet; zu diesem Zeitpunkt gibt daher das Steuerelement 17 automatisch ein Signal, welches den Rührermotor 6 umschaltet auf die normale höhere Rührgeschwindigkeit von 180 U/min (Schritt b). Wenn der Rührer 30 eine gewisse Zeit mit voller Rührgeschwindigkeit beide Komponenten gemischt hat, wird zum Zeitpunkt t2 vom Steuerelement 17 die Vakuum-Pumpe 11 in Betrieb gesetzt, deren Funktion durch den Sensor 15 überwacht und so gesteuert wird, dass der Unterdruck gegenüber dem Umgebungsdruck etwa 500 mbar beträgt und diesen Wert nicht überschreitet. Grössere Druckdifferenzen führen, wie bereits erwähnt, zu einer unzulässig hohen Verdampfung des flüssigen Monomers, während kleinere Unterdrücke die Entgasung des Gemisches beeinträchtigen und eine optimale Entgasung verhindern. Die den Schritt c bildende Entgasung der Kartusche 4 während des Mischens dauert bis zum Zeitpunkt t3. Ist dieser erreicht, so wird die Vakuum-Pumpe automatisch abgeschaltet und die Mischstation 2 durch vom Element 17 gesteuertes Umschalten des entsprechenden Ventils 10 belüftet.

Weiterhin fordern ein akustisches Signal und eine entsprechende Befehlsanzeige im Anzeigeelement bzw. der Schrifttafel 21 zum Zeitpunkt t3 auch die Kartusche 4 -bei der vorliegenden Ausführungsform - von der Mischstation 2 zu lösen und auf die Ruhestation 3 umzusetzen. Ist dies geschehen, so wird durch Drücken der Starttaste 26 die Vakuum-Pumpe wieder in Betrieb gesetzt. Gleichzeitig wird die von der Temperatur - und der Zementmischung - abhängige Ruhe-oder Wartezeit, die bis zum Zeitpunkt einer optimalen Verarbeitbarkeit des Zementes verstreichen muss oder darf, in Minuten und Sekunden angezeigt und durch Rückwärtszählen überwacht. Diese im Schritt d der Fig. 4 dargestellte Warte-oder Ruhezeit wird - neben der laufenden Anzeige des Rückwärtszählers - durch das Piep-Element 25 akustisch und optisch, beispielsweise durch Blinken der Leuchtdiode für die Anzeige "Programm läuft" angezeigt, was z.Z. t4 im Diagramm der Fall ist. Da die Warte- oder Ruhezeit temperaturabhängig ist, ergeben sich in Fig. 4 drei verschiedene Zeiten t4h, t4m und t4n für eine relativ hohe (Th), eine mittlere (Tm) und eine niedrige Umgebungstemperatur (Tn).

Erfasst wird diese Umgebungstemperatur in einem Bereich von beispielsweise 18 bis 24° durch den Messfühler 18, der den Zeitzähler im Programm des Steuerelementes 17 entsprechend beeinflusst.

Ein Drücken der Starttaste 26 zur jeweiligen Zeit t4 bewirkt ein Aufheben des Vakuums an der Ruhestation 3 bzw. in der Kartusche 4, worauf die Kartusche 4 zur Verarbeitung entnommen werden kann. Gleichzeitig erscheint auf der Schrifttafel 21 die Anzeige "Einpresszeit" mit dem zugehörigen, wieder temperaturabhängigen Zeitintervall für eine Verarbeitung des Zementes, wobei dieses Zeitintervall wiederum durch eine Rückwärtszählung überwacht wird.

In ähnlicher Weise wie das Ende der Ruhezeiten werden die zu den Zeiten t5 ablaufenden Verarbeitungszeiten (Schritt e) optisch und akustisch angezeigt. Mit den Signalen für das Ende der Verarbeitungszeit beginnt automatisch die Anzeige und Zählung der Aushärtezeiten (Schritt f), die wiederum temperaturabhängig bei den für die verschiedenen Temperaturen eingetragenen Zeiten t6 beendet sind, was ebenfalls akustisch und durch eine Anzeige in der Schrifttafel 21 signalisiert wird. Am Ende der Aushärtezeit wird das Gerät 1 automatisch abgeschaltet.

Wie bereits angedeutet, hat die Form des Rührers 30 (Fig. 5) für ein einwandfreies Mischen und für eine gute und reproduzierbare Durchmischung beider Komponenten eine erhebliche Bedeutung. Experimentell hat sich dabei eine Rührerform als besonders geeignet herausgestellt, bei der der Rührer 30 als Korkenzieher die axiale Länge der Kartusche oder des Spritzenzylinders 4 durchsetzt und in seinem Durchmesser möglichst nahe an die Wand des Zylinders 4 heranreicht, ohne diesen zu berühren. Das freie Ende des Rührers 30 bildet zunächst einen horizontalen Aufnehmer 31, der V-förmig (Fig. 6) die Mittelachse 32 des Zylinders 4 umfasst und nahe dem Boden 7 der Kartusche 4 verläuft.

Der Aufnehmer 31 geht über in einen Abreisshaken 33, der sich mindestens annähernd in einer zur Achse des Rührers 30 parallelen Ebene 35 erstreckt, die durch den Abreisshaken selbst und den ihn erzeugenden V-Arm 34 definiert ist. Der Abreisshaken 33 bildet mit dem horizontalen Boden 7 etwa einen Winkel von 60° und endet vor der achsparallelen Mittelebene 36 des Rührers 30.

Der Aufnehmer 31 hat eine Schaufelwirkung und ermöglicht die Aufnahme des Knochenzements im Randzonenbereich der Kartusche 4; er hat das Bestreben, den Knochenzement in obere Bereiche der Kartusche 4 zu befördern. Die dadurch erzeugte, aber unerwünschte Bildung einer homogenen, sich mitdrehenden Zementsäule wird durch den Abreisshaken 33 immer wieder daran gehindert, sich mit dem Rührer 30 in gleicher Geschwindigkeit mit zu drehen, weil der Abreisshaken 33 ein Zusammenbacken des Knochenzementes zu dieser kompakten Säule verhindert; durch seinen exzentrischen Lauf wird eine sich bildende Säule immer wieder abgerissen, wodurch ein homogenes Mischen des gesamten Knochenzementes erfolgt.

## Ansprüche

1. Gerät zum Steuern und Ueberwachen der Zeitintervalle beim Mischen und Verarbeiten von Knochenzement, welches Gerät eine Mischvorrichtung mit mindestens einem als Spritzenzylinder ausgebildeten Mischbehälter und, durch je einen Motor angetrieben, einen Rührer sowie eine Vakuumpumpe enthält, gekennzeichnet durch ein Steuerelement (17), das über einen Zeitzähler den Ablauf des Mischens steuert und mindestens einzelne Zeitintervalle (t) beim Verarbeiten des Knochenzementes überwacht, wobei mindestens das Ende des Mischens und mindestens einzelner überwachter Zeitintervalle durch akustische (25) und/oder optische (20, 21) Signale angezeigt wird.

2. Gerät nach Anspruch 1, gekennzeichnet durch einen Messfühler (18) für die Umgebungstemperatur, der die Dauer einzelner dieser Zeitintervalle (t4-t3; t5-t4; t6-t5) temperaturabhängig verändert.

3. Gerät nach Anspruch 1 oder 2, dadurch gekennzeichnet, dass die Dauer mindestens der temperaturabhängigen Zeitintervalle (t4-t3; t5-t4; t6-t5) für unterschiedlich zusammengesetzte Knochenzemente veränderbar ist.

4. Gerät nach einem der Ansprüche 1 bis 3, dadurch gekennzeichnet, dass der Rührermotor (6) von einer niedrigen auf höhere Rührgeschwindigkeiten umschaltbar ist.

5. Gerät nach einem der Ansprüche 1 bis 4, dadurch gekennzeichnet, das der Rührermotor (6) in seiner Drehrichtung umschaltbar ist.

6. Gerät nach einem der Ansprüche 1 bis 5, dadurch gekennzeichnet, dass das Vakuum durch einen Drucksensor (15) während der einzelnen Verarbeitungsphasen auf einer konstanten Druckdifferenz zum Umgebungsdruck gehalten ist.

7. Gerät nach einem der Ansprüche 1 bis 6, dadurch gekennzeichnt, dass der Rührer (30) eine den Spritzenzylinder (4) in axialer Richtung mindestens teilweise ausfüllende, korkenzieherähnliche Form hat, deren Aussendurchmesser an den Innendurchmesser des Mischzylinders (4) angepasst ist.

8. Gerät nach Anspruch 7, dadurch gekennzeichnet, dass der Rührer (30) an seinem freien Ende in einer horizontalen Aufnehmer (31) übergeht, der V-förmig die Mittelachse (32) des Zylinders (4) umfasst, ehe er in einem in den Korkenzieher hinein gerichteten Abreisshaken (33) endet.

9. Gerät nach Anspruch 7 oder 8, dadurch gekennzeichnet, dass der Abreisshaken (33) mindestens annähernd mit dem ihn erzeugenden V-Arm (34) in einer zur Rührerachse parallelen Ebene (35) verläuft.

10. Gerät nach einem der Ansprüche 7 bis 9, dadurch gekennzeichnet, dass der Abreisshaken (33) aus der Horizontalen heraus unter einem Winkel von mindestens annähernd 60° nach oben gerichtet ist.

11. Gerät nach einem der Ansprüche 7 bis 10, dadurch gekennzeichnet, dass der Abreisshaken (33) vor der achsparallelen Mittelebene (36) des Rührers (30) endet.

F I G. 1

F I G. 2

Fig. 3

FIG. 4

0 290 746

FIG. 6

FIG. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 029 049 (KEMPER)<br>* Zusammenfassung; Figuren *<br>--- | 1,3-5 | B 01 F 15/00 |
| Y | EP-A-0 112 619 (MASTERMIX)<br>* Zusammenfassung; Figur *<br>--- | 1,3-5 | |
| A | US-A-4 201 483 (FRANZKE)<br>* Zusammenfassung *<br>--- | 6 | |
| A | US-A-3 018 666 (HOFFMANN)<br>--- | | |
| A | US-A-4 403 867 (DUKE)<br>--- | | |
| A | US-A-4 340 310 (CLARK)<br>----- | | |

RECHERCHIERTE
SACHGEBIETE (Int. Cl.4)

B 01 F
B 28 C
B 29 B

**Der vorliegende Recherchenbericht wurde für alle Patentansprüche erstellt**

| Recherchenort | Abschlußdatum der Recherche | Prüfer |
|---|---|---|
| DEN HAAG | 31-08-1988 | PEETERS S. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.............................................................................
& : Mitglied der gleichen Patentfamilie, übereinstimmendes
    Dokument

EPO FORM 1503 03.82 (P0403)